# EUROPEAN PATENT APPLICATION

(11) **EP 2 757 363 A1**
(43) Date of publication of application: **23.07.2014**
(21) Application number: 12831848.2
(22) Date of filing: 02.08.2012
(51) Int. Cl.: G01N 21/27, G01N 21/03

(54) **SPR SENSOR CELL AND SPR SENSOR**

(30) Priority: 15.09.2011 JP 2011201299
(71) Applicant: Nitto Denko Corporation, Ibaraki-shi, Osaka 567-8680 (JP)
(72) Inventor: KONTANI, Tomohiro, Ibaraki-shi Osaka 567-8680 (JP)
(74) Representative: Alt, Michael
(86) International application number: PCT/JP2012/069712
(87) International publication number: WO 2013/038830

(57) **Abstract**

Provided are an SPR sensor cell having very excellent detection sensitivity and an SPR sensor. The SPR sensor cell includes a detection unit; and a sample mounting portion adjacent to the detection unit, wherein: the detection unit includes an under clad layer, a core layer formed so that at least a part thereof is adjacent to the under clad layer, a metal layer covering the core layer, and a coat layer covering the metal layer; the coat layer is formed of a material having a band gap (Eg) of more than 1.0 eV and less than 7.0 eV; and a refractive index of the coat layer is higher than a refractive index of the core layer.

## Description

### Technical Field

The present invention relates to an SPR sensor cell and an SPR sensor. More specifically, the present invention relates to an SPR sensor cell including an optical waveguide and an SPR sensor.

### Background Art

Hitherto, in the fields of chemical analysis, biochemical analysis, and the like, a surface plasmon resonance (SPR) sensor including an optical fiber has been used. In the SPR sensor including an optical fiber, a metal thin film is formed on an outer circumferential surface of a tip end portion of the optical fiber, and an analysis sample is fixed to the optical fiber into which light is guided. Among the light to be guided, light having a particular wavelength generates surface plasmon resonance in the metal thin film, and light intensity thereof is attenuated. In such an SPR sensor, the wavelength of the light generating surface plasmon resonance generally varies depending on a refractive index of an analysis sample to be fixed to the optical fiber. Therefore, if a wavelength at which light intensity is attenuated after the generation of surface plasmon resonance is measured, the wavelength of the light generating surface plasmon resonance can be identified. Further, if a change in the wavelength at which light intensity is attenuated is detected, it can be confirmed that the wavelength of the light generating surface plasmon resonance has changed, and hence a change in refractive index of the analysis sample can be confirmed. As a result, such an SPR sensor can be used for various chemical analyses and biochemical analyses such as measurement of a sample concentration and detection of an immunoreaction.

For example, in the case where the sample is a solution, the refractive index of the sample (solution) depends on a concentration of the solution. Therefore, the concentration of the sample can be detected by measuring the refractive index of the sample (solution) with the SPR sensor in which the sample (solution) is in contact with the metal thin film, and further, it can be confirmed that the concentration of the sample (solution) has changed by confirming a change in the refractive index. In analysis of the immunoreaction, for example, an antibody is fixed onto the metal thin film of the optical fiber in the SPR sensor through intermediation of a dielectric film, an analyte is brought into contact with the antibody, and surface plasmon resonance is generated. In this case, if the antibody and the analyte perform the immunoreaction, the refractive index of the sample changes. Therefore, it can be determined that the antibody and the analyte have performed the immunoreaction by confirming that the refractive index of the sample has changed before and after the contact between the antibody and the analyte.

In the SPR sensor including an optical fiber, the tip end portion of the optical fiber has a fine cylindrical shape, and hence there is a problem in that it is difficult to form the metal thin film and fix an analysis sample to the optical fiber. In order to solve the problem, for example, there has been proposed an SPR sensor cell including a core through which light is transmitted and a clad covering the core, in which a through-hole extending to a surface of the core is formed at a predetermined position of the clad, and a metal thin film is formed on the surface of the core at a position corresponding to the through-hole (for example, Patent Literature 1). In such an SPR sensor cell, it is easy to form the metal thin film for generating surface plasmon resonance on the surface of the core and fix the analysis sample onto the surface.

However, in recent years, in chemical analysis and biochemical analysis, there is an increasing demand for detection of a minute change and/or a trace amount of component, and thus further enhancement of detection sensitivity of the SPR sensor cell is being demanded.

### Citation List

### Patent Literature

[PTL 1] JP 2000-19100 A

### Summary of Invention

### Technical Problem

The present invention has been made in view of solving the conventional problem, and an object of the present invention is to provide an SPR sensor cell having very excellent detection sensitivity and an SPR sensor.

### Solution to Problem

According to one embodiment of the present invention, there is provided an SPR sensor cell, including: a detection unit; and a sample mounting portion adjacent to the detection unit, in which: the detection unit includes an under clad layer, a core layer that is formed so that at least a part thereof is adjacent to the under clad layer, a metal layer covering the core layer, and a coat layer covering the metal layer; the coat layer is formed of a material having a band gap (Eg) of more than 1.0 eV and less than 7.0 eV; and the refractive index of the coat layer is higher than the refractive index of the core layer.

In a preferred embodiment, the material for forming the coat layer includes at least one material selected from silicon, zinc selenide, gallium arsenide, aluminum nitride, and N,N'-di(1-naphthyl)-N,N'-diphenylbenzidine.

In a preferred embodiment, the refractive index of the coat layer is 1.60 or more.

In a preferred embodiment, the coat layer has an extinction coefficient of 1.80×10⁻² or less.

In a preferred embodiment, the coat layer has a thickness of 5 nm to 260 nm.

According to another aspect of the present invention, there is provided an SPR sensor. The SPR sensor includes the SPR sensor cell.

### Advantageous Effects of Invention

According to one embodiment of the present invention, the SPR sensor cell having extremely excellent detection sensitivity and the SPR sensor can be provided by covering the core layer of an optical waveguide serving as the detection unit with the metal layer, and further covering the metal layer with the coat layer formed of a material having a band gap (Eg) of more than 1.0 eV and less than 7.0 eV.

### Brief Description of Drawings

FIG. **1** is a schematic perspective view illustrating an SPR sensor cell according to a preferred embodiment of the present invention.
FIG. **2** is a schematic sectional view of the SPR sensor cell illustrated in FIG. **1****.**
FIGS. **3** are schematic sectional views illustrating an example of a method of producing an SPR sensor cell of the present invention.
FIG. **4** is a schematic sectional view illustrating an SPR sensor according to a preferred embodiment of the present invention.

### Description of Embodiments

### A. SPR sensor cell

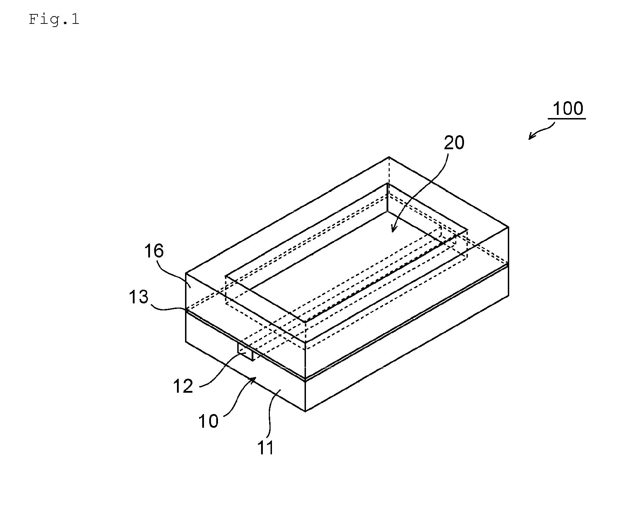

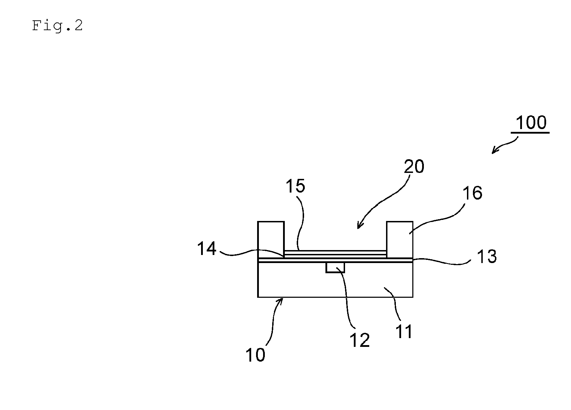

FIG. **1** is a schematic perspective view illustrating an SPR sensor cell according to a preferred embodiment of the present invention. FIG. **2** is a schematic sectional view of the SPR sensor cell illustrated in FIG. **1****.** Note that, when a direction is mentioned in the following description of the SPR sensor cell, an upper side of the drawing is defined as an upper side, and a lower side of the drawing is defined as a lower side.

As illustrated in FIGS. **1** and **2**, an SPR sensor cell **100** is formed in a shape of a bottomed frame having a substantially rectangular shape in a plan view, and includes a detection unit **10** and a sample mounting portion **20** adjacent to the detection unit **10.** The detection unit **10** is provided so as to detect the state of a sample to be mounted in the sample mounting portion **20** and/or a change therein. The detection unit **10** includes an optical waveguide. In the illustrated embodiment, the detection unit **10** is substantially formed of the optical waveguide. Specifically, the detection unit **10** includes an under clad layer **11,** a core layer **12,** a protective layer **13,** a metal layer **14,** and a coat layer **15.** The sample mounting portion **20** is defined by an over clad layer **16.** The protective layer **13** may be omitted depending on the purpose. The over clad layer **16** may also be omitted as long as the sample mounting portion **20** can be provided appropriately. In the sample mounting portion **20,** a sample (for example, a solution or powder) to be analyzed is mounted so as to come into contact with the detection unit (substantially, the coat layer).

The under clad layer **11** is formed in a shape of a plate having a substantially rectangular shape in a plan view, with a predetermined thickness. The thickness of the under clad layer (thickness from an upper surface of the core layer) is, for example, 5 µm to 400 µm.

The core layer **12** is formed substantially in a square column shape (more specifically, a rectangular shape in a cross-section flattened in a width direction) extending in a direction orthogonal to both a width direction (right and left direction of the drawing surface of FIG. **2****)** and a thickness direction of the under clad layer **11,** and is buried in an upper end portion substantially at the center of the width direction of the under clad layer **11.** The direction in which the core layer **12** extends serves as a direction in which light is propagated in the optical waveguide. The thickness of the core layer is, for example, 5 µm to 200 µm, and the width of the core layer is, for example, 5 µm to 200 µm.

The core layer **12** is disposed so that the upper surface thereof is exposed from the under clad layer **11.** Preferably, the core layer **12** is disposed so that the upper surface thereof is flush with an upper surface of the under clad layer **11.** The metal layer **14** can be disposed efficiently only on an upper side of the core layer **12** by disposing the core layer so that the upper surface thereof is flush with the upper surface of the under clad layer. Further, the core layer **12** is disposed so that both end surfaces thereof in the extending direction are flush with both end surfaces of the under clad layer in the extending direction.

The refractive index of the core layer **12** is preferably 1.33 to 1.59. The relationship between the refractive index of the core layer **12** and the refractive index of a coat layer described later can be made appropriate by setting the refractive index of the core layer to 1.59 or less, with the result that the detection sensitivity can be remarkably enhanced. When the refractive index of the core layer is 1.33 or more, SPR can be excited even in an aqueous solution-based sample (refractive index of water: 1.33), and a general-purpose material can be used. Note that, the "refractive index" as used herein refers to a refractive index at a wavelength of 850 nm unless otherwise specified.

The refractive index of the core layer **12** is higher than that of the under clad layer **11.** The difference between the refractive index of the core layer and that of the under clad layer is preferably 0.010 or more, more preferably 0.020 or more. When the difference between the refractive index of the core layer and that of the under clad layer is in such a range, the optical waveguide of the detection unit can be set to a so-called multimode. Thus, the amount of light transmitted through the optical waveguide can be increased, and as a result, the S/N ratio can be enhanced.

As a material for forming the core layer **12,** any suitable material can be used as long as the effect of the present invention is obtained. Specific examples thereof include a fluorine resin, an epoxy resin, a polyimide resin, a polyamide resin, a silicone resin, an acrylic resin, and modified products thereof (for example, a fluorene-modified product, a heavy hydrogen-modified product, and a fluorine-modified product in the case of the resins other than the fluorine resin). These resins may be used alone or in combination. These resins each can be used as a photosensitive material preferably by being blended with a photosensitizing agent. The under clad layer **11** can be formed of a material that is similar to that for forming the core layer and is adjusted so that the refractive index thereof becomes lower than that of the core layer.

The protective layer **13** is formed as a thin film in the same shape as that of the under clad layer in a plan view so as to cover all the upper surfaces of the under clad layer **11** and the core layer **12,** as necessary. By providing the protective layer **13,** for example, in the case where a sample is a liquid, the core layer and/or the clad layer can be prevented from being swollen with the sample. As a material for forming the protective layer **13,** for example, silicon dioxide and aluminum oxide may be utilized. The thickness of the protective layer **13** is preferably 1 nm to 100 nm, more preferably 5 nm to 20 nm.

As illustrated in FIG. **2****,** the metal layer **14** is formed so as to uniformly cover the upper surface of the core layer **12** through intermediation of the protective layer **13.** In this case, as necessary, an easy-adhesion layer (not shown) may be provided between the protective layer **13** and the metal layer **14.** By forming the easy-adhesion layer, the protective layer **13** and the metal layer **14** can be fixed to each other firmly. The core layer **12** may be directly covered with the metal layer **14** without providing the protective layer **13.**

As a material for forming the metal layer **14,** gold, silver, platinum, copper, aluminum, and alloys thereof may be utilized. The metal layer **14** may be a single layer or may have a laminate structure of two or more layers. The thickness (total thickness of all the layers in the case of the laminate structure) of the metal layer **14** is preferably 40 nm to 70 nm, more preferably 50 nm to 60 nm.

As a material for forming the easy-adhesion layer, chromium or titanium may typically be utilized. The thickness of the easy-adhesion layer is preferably 1 nm to 5 nm.

As illustrated in FIG. **2****,** the coat layer **15** is formed so as to cover the metal layer **14.** The coat layer **15** is formed of a material having a band gap (Eg) of more than 1.0 eV and less than 7.0 eV, and is preferably formed of a material having a band gap (Eg) of preferably more than 1.0 eV and less than 5.5 eV, more preferably more than 1.0 eV and less than 3.5 eV. When the coat layer is formed of such material, the refractive index of the coat layer can be made sufficiently high, and its extinction coefficient can be made sufficiently small. As a result, an excellent S/N ratio and detection sensitivity can be obtained. The band gap (Eg) can be determined by, for example, measuring an optical absorption spectrum through use of a spectroscopic method. Note that, in this description, the band gap (Eg) is a value at 27°C.

The material for forming the coat layer is not limited as long as the material has a band gap within the above-mentioned range. For example,aninorganicsemiconductor material,anorganic semiconductor material, or an extrinsic semiconductor material thereof may be utilized. Specific examples of the inorganic semiconductor material include: element semiconductors such as silicon and carbon; and compound semiconductors such as zinc selenide, gallium arsenide, and aluminum nitride. In addition, specific examples of the organic semiconductor material include: low-molecular-weight materials such as arylamine derivatives (such as N,N'-di(1-naphthyl)-N,N'-diphenylbenzidine) and a quinolinol metal complex; and high-molecular-weight materials such as polyacetylene and polyaniline. Examples of the extrinsic semiconductor material include semiconductor materials each obtained by adding a dopant to any of the inorganic semiconductor materials and the organic semiconductor materials. The kind and addition amount of the dopant can be appropriately set depending on a desired band gap and the like. In one embodiment, as the material for forming the coat layer, silicon, zinc selenide, gallium arsenide, aluminum nitride, and N,N'-di(1-naphthyl)-N,N'-diphenylbenzidine may be preferably utilized. This is because a coat layer having an appropriate refractive index and extinction coefficient can be formed.

The refractive index of the coat layer **15** is higher than the refractive index of the core layer **12.** Specifically, the refractive index of the coat layer is preferably 1.60 or more, more preferably 2.00 or more, still more preferably 2.50 or more. When the refractive index of the coat layer is less than 1.60, sufficient detection sensitivity may not be obtained.

The extinction coefficient of the coat layer **15** is preferably 1.80×10⁻² or less, more preferably 1.50×10⁻² or less. The lower limit of the extinction coefficient is preferably 0 (zero). When the extinction coefficient is in such range, an SPR peak can be expressed satisfactorily.

The thickness of the coat layer **15** is preferably 5 nm to 260 nm, more preferably 10 nm to 100 nm, still more preferably 15 nm to 50 nm. In the case where the thickness is less than 5 nm, detection sensitivity may not be enhanced sufficiently. When the thickness is more than 260 nm, SPR is influenced by the absorption of the core layer due to the excitation wavelength increased too much, with the result that appropriate detection may not be performed.

As illustrated inFIG. **1,** the over clad layer **16** is formed in the shape of a frame having a rectangular shape in a plan view so that an outer circumference of the over clad layer **16** becomes substantially flush with an outer circumference of the under clad layer **11** in a plan view, on the upper surfaces of the under clad layer **11** and the core layer **12** (upper surface of the protective layer **13** in the illustrated example). A portion surrounded by the upper surfaces of the under clad layer **11** and the core layer **12** (upper surface of the protective layer **13** in the illustrated example) and the over clad layer **16** is partitioned as the sample mounting portion **20.** By mounting a sample in the partitioned portion, the metal layer of the detection unit **10** and the sample come into contact with each other so that detection can be performed. Further, by forming such a partitioned portion, a sample can be easily mounted on the surface of the metal layer, and hence the operability can be enhanced.

As a material for forming the over clad layer **16,** for example, the materials for forming the core layer and the under clad layer, and silicone rubber may be utilized. The thickness of the over clad layer is preferably 5 µm to 2, 000 µm, more preferably 25 µm to 200 µm. The refractive index of the over clad layer is preferably lower than that of the core layer. In one embodiment, the refractive index of the over clad layer is equal to that of the under clad layer. Note that, in the case of forming a protective layer having a refractive index lower than that of the core layer, the refractive index of the over clad layer is not necessarily required to be lower than that of the core layer.

Although the SPR sensor cell according to the preferred embodiment of the present invention has been described, the present invention is not limited thereto. For example, in the relationship between the core layer and the under clad layer, at least a portion of the core layer has only to be adjacent to the under clad layer. For example, although a configuration in which the core layer is buried in the under clad layer is described in the above-mentioned embodiment, the core layer may be provided so as to pass through the under clad layer. Alternatively, the core layer may be formed on the under clad layer so that a predetermined portion of the core layer is surrounded by the over clad layer.

Further, the number of core layers in the SPR sensor cell may be changed depending on the purpose. Specifically, a plurality of the core layers may be formed at a predetermined interval in the width direction of the under clad layer. With such a configuration, a plurality of samples can be analyzed simultaneously, and hence analysis efficiency can be enhanced. As the shape of the core layer, any suitable shape (for example, a semicircular column shape or a convex column shape) can be adopted depending on the purpose.

Further, a lid may be provided on an upper portion of the SPR sensor cell **100** (sample mounting portion **20**)**.** With such a configuration, a sample can be prevented from coming into contact with ambient air. Further, in the case where the sample is a solution, a change in concentration caused by evaporation of a solvent can be prevented. In the case of providing a lid, an injection port for injecting a liquid sample into the sample mounting portion and a discharge port for discharging the liquid sample from the sample mounting portion may be provided. With such a configuration, the sample can be allowed to flow and to be supplied to the sample mounting portion continuously, and hence the characteristics of the sample can be measured continuously.

The above-mentioned embodiments may be combined appropriately.

### B. Method of producing SPR sensor cell

The SPR sensor cell of the present invention can be produced by any suitable method. As an example, a method of producing an SPR sensor cell adopting a stamper system as a method of forming a core layer on an under clad layer is described. As the method of forming a core layer on an under clad layer, for example, photolithography (direct exposure system) using a mask as well as the stamper system may be utilized. Note that, the photolithography is well known.

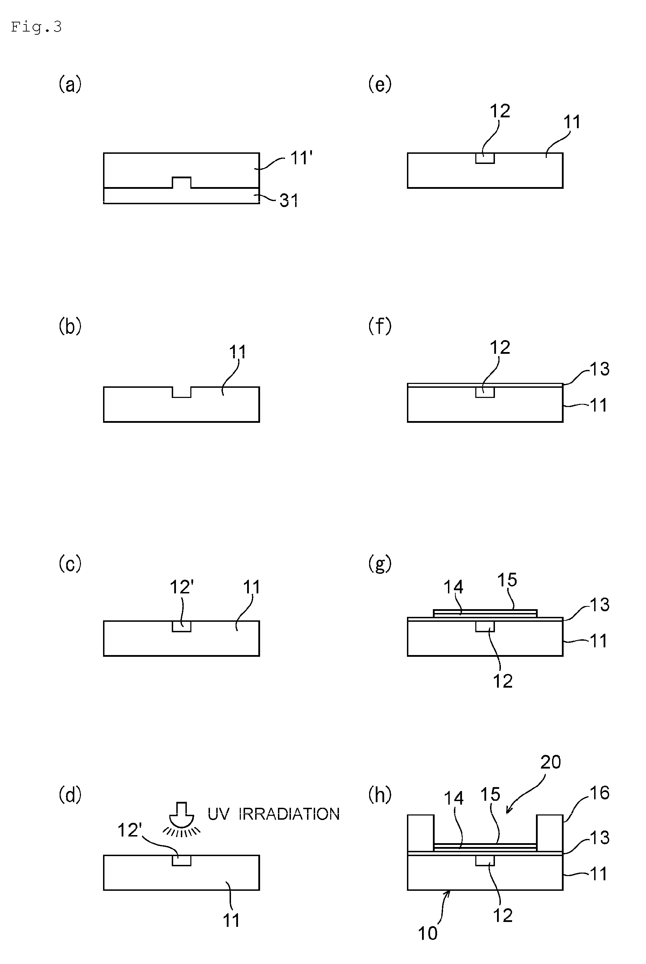

FIGS. **3(a)** to **3(h)** are schematic sectional views illustrating the method of producing an SPR sensor cell adopting a stamper system as a method of forming a core layer on an under clad layer. First, as illustrated in FIG. **3(a)****,** a material **11'** for forming an under clad layer is applied to a die 31 having a protrusion corresponding to a core layer formation portion of the under clad layer, and the material for forming an under clad layer applied to the die is irradiated with ultraviolet rays to cure the material. The irradiation conditions of ultraviolet rays can be set appropriately depending on the kind of the material for forming an under clad layer. The under clad layer **11** is formed by curing the material for forming an under cladlayer. Further, as illustrated in FIG. **3(b)****,** the under clad layer **11** thus formed is peeled from the die.

Then, as illustrated in FIG. **3(c)****,** a groove portion of the under clad layer **11** is filled with a material **12'** for forming a core layer. Further, of the material for forming a core layer filling the groove portion of the under clad layer, an excess material for forming a core layer overflowed the concave groove is scraped with a scraper in accordance with a method of producing a polymer optical waveguide described in JP 09-281351 A. Thus, the core layer and the under clad layer can be rendered flush with each other. Further, as illustrated in FIG. **3(d)****,** the material **12'** for forming a core layer filling the groove portion is irradiated with ultraviolet rays to cure the material. The irradiation conditions of ultraviolet rays can be set appropriately depending on the kind of the material for forming a core layer. As necessary, the material for forming a core layer may be heated. The heating may be performed before or after the irradiation with ultraviolet rays, or simultaneously with the irradiation with ultraviolet rays. The heating conditions can be set appropriately depending on the kind of the material for forming a core layer. By curing the material for forming a core layer, as illustrated in FIG. **3(e)****,** the core layer **12** buried in the under clad layer **11** is formed.

As necessary, as illustrated in FIG. **3(f)****,** the protective layer **13** is formed on the under clad layer **11** and the core layer **12.** The protective layer is formed, for example, by subjecting a material for forming a protective layer to sputtering or vapor deposition. In the case of forming the protective layer, preferably, an easy-adhesion layer (not shown) is formed on the protective layer. The easy-adhesion layer is formed, for example, by subjecting chromium or titanium to sputtering.

Next, as illustrated in FIG. **3(g)****,** the metal layer **14** is formed on the protective layer **13** (upper surfaces of the core layer and the under clad layer in the case where the protective layer is not formed) so as to cover the core layer **12,** and the coat layer **15** is formed so as to cover the metal layer **14.** Specifically, the metal layer **14** is formed, for example, by subjecting a material for forming a metal layer to vacuum deposition, ion plating, or sputtering through a mask having a predetermined pattern. The coat layer **15** is formed by subjecting a material for forming the coat layer to vacuum deposition, chemical vapor deposition, ion plating, or sputtering through a mask having a pattern similar to that in the case of forming the metal layer. In the case of using the organic semiconductor material, the coat layer can be formed by applying the organic semiconductor material. The metal layer **14** and the coat layer **15** may be formed continuously.

Finally, as illustrated in FIG. **3(h)**, the over clad layer **16** having the predetermined frame shape is formed. The over clad layer **16** can be formed by any suitable method. The over clad layer **16** can be formed, for example, by disposing a die having the predetermined frame shape on the protective layer **13,** filling the die with varnish of a material for forming an over clad layer, drying the varnish, curing the varnish as necessary, and finally removing the die. In the case of using a photosensitive material, the over clad layer **16** can be formed by applying the varnish over the entire surface of the protective layer **13,** drying the varnish, and then exposing the varnish to light through a photomask having a predetermined pattern, followed by development.

Accordingly, the SPR sensor cell can be produced by the method described above.

### C. SPR sensor

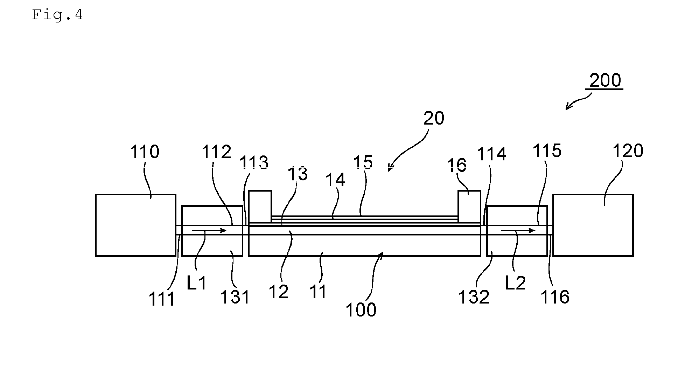

FIG. **4** is a schematic sectional view illustrating an SPR sensor according to a preferred embodiment of the present invention. An SPR sensor **200** includes the SPR sensor cell **100,** a light source **110,** and an optical measuring instrument **120.** The SPR sensor cell **100** is the SPR sensor cell of the present invention described in the above-mentioned sections A and B.

As the light source **110,** any suitable light source can be adopted. Specific examples of the light source include a white light source and a monochromatic light source. The optical measuring instrument **120** is connected to any suitable arithmetic processing device, and enables accumulation, display and processing of data.

The light source **110** is connected to a light source side optical fiber **112** through a light source side optical connector **111.** The light source side optical fiber **112** is connected to one side end portion in a propagation direction of the SPR sensor cell **100** (core layer **12**) through a light source side fiber block **113.** A measuring instrument side optical fiber **115** is connected to the other side end portion in the propagation direction of the SPR sensor cell **100** (core layer **12**) through a measuring instrument side fiber block **114.** The measuring instrument side optical fiber **115** is connected to the optical measuring instrument **120** through a measuring instrument side optical connector **116.**

The SPR sensor cell **100** is fixed by any suitable sensor cell fixing device (not shown). The sensor cell fixing device is movable in a predetermined direction (for example, a width direction of the SPR sensor cell), and thus the SPR sensor cell can be disposed at a desired position.

The light source side optical fiber **112** is fixed by a light source side optical fiber fixing device **131,** and the measuring instrument side optical fiber **115** is fixed by a measuring instrument side optical fiber fixing device **132.** The light source side optical fiber fixing device **131** and the measuring instrument side optical fiber fixing device **132** are each fixed to any suitable six-axis movable stage (not shown) so as to be movable in the propagation direction of the optical fiber, width direction (direction orthogonal to the propagation direction in a horizontal direction) and thickness direction (direction orthogonal to the propagation direction in a perpendicular direction), and rotatable about axes in the above-mentioned respective directions.

In the SPR sensor as described above, the light source **110,** the light source side optical fiber **112,** the SPR sensor cell **100** (core layer **12**), the measuring instrument side optical fiber **115,** and the optical measuring instrument **120** can be arranged on one axis, and light can be guided from the light source **110** so as to be transmitted therethrough.

An example of the manner of use of such an SPR sensor is described below.

First, a sample is mounted on the sample mounting portion **20** of the SPR sensor cell **100,** and the sample and the metal layer **14** are brought into contact with each other through intermediation of the coat layer **15**. Then, predetermined light from the light source **110** is guided to the SPR sensor cell **100** (core layer **12)** through the light source side optical fiber **112** (see an arrow L1 of FIG. **4**)**.** The light guided to the SPR sensor cell **100** (core layer **12**) is transmitted through the SPR sensor cell **100** (core layer **12**) while repeating total internal reflection in the core layer **12,** and part of the light enters the metal layer **14** on an upper surface of the core layer **12** and is attenuated by surface plasmon resonance. The light transmitted through the SPR sensor cell **100** (core layer **12)** is guided to the optical measuring instrument **120** through the measuring instrument side optical fiber **115** (see an arrow L2 of FIG. **4**). That is, in the SPR sensor **200,** the intensity of light having a wavelength generating surface plasmon resonance in the core layer **12** is attenuated in the light guided to the optical measuring instrument **120.** The wavelength of light generating surface plasmon resonance depends on, for example, the refractive index of the sample brought into contact with the metal layer **14** (substantially, the coat layer **15**)**.** Therefore, by detecting the attenuation of the light intensity of the light guided to the optical measuring instrument **120,** a change in refractive index of the sample can be detected.

For example, in the case of using a white light source as the light source **110,** a change in refractive index of the sample can be confirmed by measuring the wavelength of light whose light intensity is attenuated after the transmission through the SPR sensor cell **100** (wavelength of light generating surface plasmon resonance) with the optical measuring instrument **120** and detecting a change in wavelength of the light whose light intensity is attenuated. Further, for example, in the case of using a monochromatic light source as the light source **110,** a change in wavelength of light generating surface plasmon resonance can be confirmed and a change in refractive index of the sample can be confirmed by measuring a change (attenuation degree) in light intensity of monochromatic light after the transmission through the SPR sensor cell **100** with the optical measuring instrument **120** and detecting a change in attenuation degree.

As described above, such an SPR sensor cell can be used, for example, for various chemical analyses and biochemical analyses such as the measurement of a sample concentration and the detection of an immunoreaction, based on a change in refractive index of the sample. More specifically, for example, in the case where the sample is a solution, the refractive index of the sample (solution) depends on the concentration of the solution, and hence the concentration of the sample can be measured by detecting the refractive index of the sample. Further, a change in concentration of the sample can be confirmed by detecting a change in refractive index of the sample. Further, for example, in the detection of an immunoreaction, an antibody is fixed onto the metal layer **14** (substantially, the coat layer **15**) of the SPR sensor cell **100** through intermediation of a dielectric film, and an analyte is brought into contact with the antibody. If the antibody and the analyte perform an immunoreaction, the refractive index of the sample changes. Therefore, it can be determined that the antibody and the analyte have performed an immunoreaction by detecting a change in refractive index of the sample before and after the contact between the antibody and the analyte.

### Examples

The present invention is hereinafter described specifically by way of Examples. However, the present invention is not limited thereto. Note that, unless otherwise specified, the measurement wavelength for a refractive index is 850 nm and each measurement wavelength for an absorption coefficient and an extinction coefficient is 1,200 nm in Examples and Comparative Examples.

### <Example 1>

An optical waveguide was formed through use of the stamper system as illustrated in FIGS. **3(a)** to **3(e)****.** Specifically, a fluorine-based UV curable resin ("OP38Z" (trade name) manufactured by DIC Corporation) which was a material for forming an under clad layer was applied to a die having a protrusion corresponding to a core layer formation portion of an under clad layer, and the resin was cured with ultraviolet rays to form an under clad layer. The refractive index of the under clad layer thus obtained was 1.372. The under clad layer had a length of 80 mm, a width of 80 mm, and a thickness of 150 µm, and a groove portion for forming a core layer having a width of 50 µm and a thickness (depth) of 50 µm was formed in the under clad layer. After the under clad layer was peeled from the die, the groove portion was filled with a fluorine-based UV-curable resin ("OP40Z" (trade name) manufactured by DIC Corporation) serving as a material for forming a core layer to form a core layer. The refractive index of the core layer thus formed was 1.399 (wavelength: 850 nm), and the absorption coefficient thereof was 2.90×10⁻² (mm⁻¹). Note that, as for the refractive index, a film of the material for forming a core layer having a thickness of 10 µm was formed on a silicon wafer, and the refractive index of the film at a wavelength of 850 nm was measured through use of a prism coupler refractive index measurement device. As for the absorption coefficient, a film of the material for forming a core layer having a thickness of 50 µm was formed on a glass substrate, and the absorption coefficient of the film at a wavelength of 1,200 nm was measured through use of a spectrophotometer. As described above, a buried-type optical waveguide film was produced.

Then, SiO₂ was sputtered onto the entire surface of an upper surface (core layer exposed surface) of the optical waveguide film thus obtained to form a protective layer (thickness: 10 nm). The optical waveguide film with the protective layer formed thereon was subjected to die cutting to a length of 20 mm and a width of 22.25 mm. After that, chromium and gold were sputtered onto the cut optical waveguide film in the stated order through a mask with an opening having a length of 6 mm and a width of 1 mm, and thus an easy-adhesion layer (thickness: 1 nm) and a metal layer (thickness: 50 nm) were formed in the stated order so as to cover the core layer through intermediation of the protective layer. Further, silicon (Si) was sputtered through a mask similar to that described above to form an amorphous silicon film as a coat layer (thickness: 20 nm, band gap: 1.1 eV) covering the metal layer. The refractive index of the coat layer thus obtained was 4. 1, and the extinction coefficient thereof was 1.1×10⁻². The thickness of the coat layer was measured by observing a cross-section of the coat layer with a transmission electron microscope (TEM). As for the refractive index, a silicon film having a thickness of 100 nm was formed on a glass substrate, and the refractive index of the silicon film at a wavelength of 850 nm was measured through use of a spectroscopic ellipsometer. As for the extinction coefficient, a silicon film having a thickness of 100 nm was formed on a glass substrate, and the extinction coefficient of the silicon film at a wavelength of 1,200 nm was calculated through use of a spectroscopic ellipsometer.

Finally, a frame-shaped over clad layer was formed by a method similar to that for forming the under clad layer, through use of the same material as that for forming the under clad layer. Accordingly, the SPR sensor cell as illustrated in FIGS. **1** and **2** was produced.

The SPR sensor cell obtained as described above, a halogen light source ("HL-2000-HP" (trade name) manufactured by Ocean Optics, Inc.), and a spectroscope ("USB4000"and"NIRQuest512" (trade names) manufactured by Ocean Optics, Inc.) were arranged on one axis and connected to each other to produce an SPR sensor as illustrated in FIG. **4****.** 40 µL each of four kinds of ethylene glycol aqueous solutions of different concentrations (concentration: 0 vol% (refractive index: 1.3330), 1 vol% (refractive index: 1.3340), 5 vol% (refractive index: 1.3383), and 10 vol% (refractive index: 1.3436)) were supplied to the sample mounting portion of the SPR sensor cell and subjected to measurement. Further, a transmittance spectrum was determined in the case where light intensity at each wavelength when light was transmitted through the SPR sensor cell (optical waveguide) under the condition that the sample (ethylene glycol aqueous solution) was not mounted was set to 100%, and a wavelength λmin corresponding to a minimum value of a transmittance was measured. A relationship between the refractive index of the ethylene glycol aqueous solution and the λmin was plotted on XY coordinates with the X axis representing the refractive index and the y axis representing λmin to create a calibration line, and a gradient of the calibration line was determined. A larger gradient means higher detection sensitivity. Table 1 below shows the refractive index and the extinction coefficient of the coat layer, λmin when the sample concentration is 10 vol%, and the gradient (detection sensitivity).

### <Example 2>

An SPR sensor cell and an SPR sensor were produced in the same way as in Example 1 except for forming a coat layer (thickness: 20 nm) by sputtering zinc selenide (ZnSe). The SPR sensor thus obtained was evaluated in the same way as in Example 1. Table 1 shows the results.

### <Example 3>

An SPR sensor cell and an SPR sensor were produced in the same way as in Example 1 except for forming a coat layer (thickness: 40 nm) by sputtering zinc selenide (ZnSe). The SPR sensor thus obtained was evaluated in the same way as in Example 1. Table 1 shows the results.

### <Example 4>

An SPR sensor cell and an SPR sensor were produced in the same way as in Example 1 except for forming a coat layer (thickness: 20 nm) by subjecting an arylamine derivative, N,N'-di(1-naphthyl)-N,N'-diphenylbenzidine (NPD), to vacuum deposition. The SPR sensor thus obtained was evaluated in the same way as in Example 1. Table 1 shows the results.

### <Example 5>

An SPR sensor cell and an SPR sensor were produced in the same way as in Example 1 except for forming a coat layer (thickness: 20 nm) by subjecting gallium arsenide (GaAs) to vacuum deposition. The SPR sensor thus obtained was evaluated in the same way as in Example 1. Table 1 shows the results.

### <Example 6>

An SPR sensor cell and an SPR sensor were produced in the same way as in Example 1 except for forming a coat layer (thickness: 20 nm) by sputtering aluminum nitride (AlN). The SPR sensor thus obtained was evaluated in the same way as in Example 1. Table 1 shows the results.

### <Comparative Example 1>

An SPR sensor cell and an SPR sensor were produced in the same way as in Example 1 except for not forming a coat layer. The SPR sensor thus obtained was evaluated in the same way as in Example 1. Table 1 shows the results.

### <Comparative Example 2>

An SPR sensor cell and an SPR sensor were produced in the same way as in Example 1 except for forming a coat layer (thickness: 20 nm) by sputtering chromium. The SPR sensor thus obtained was evaluated in the same way as in Example 1. Table 1 shows the results.

### <Comparative Example 3>

An SPR sensor cell and an SPR sensor were produced in the same way as in Example 1 except for forming a coat layer (thickness: 20 nm) by sputtering gallium antimonide (GaSb). The SPR sensor thus obtained was evaluated in the same way as in Example 1. Table 1 shows the results.

### <Comparative Example 4>

An SPR sensor cell and an SPR sensor were produced in the same way as in Example 1 except for forming a coat layer (thickness: 20 nm) by sputtering silicon dioxide (SiO₂). The SPR sensor thus obtained was evaluated in the same way as in Example 1. Table 1 shows the results.

**[Table 1]**

| | Coat layer | | | | | Gradient | λmin (nm) |
|---|---|---|---|---|---|---|---|
| | Material | Band gap (eV) | Thickness (nm) | Refractive index | Extinction coefficient | | |
| Example 1 | Si | 1.1 | 20 | 4.1 | 1.1×10⁻² | 13,121 | 1,475 |
| Example 2 | ZnSe | 2.7 | 20 | 2.5 | 0 | 8,670 | 1,180 |
| Example 3 | ZnSe | 2.7 | 40 | 2.5 | 0 | 12,555 | 1,412 |
| Example 4 | NPD | 3.0 | 20 | 1.8 | 0 | 7,585 | 1,011 |
| Example 5 | GaAs | 3.3 | 20 | 3.4 | 0 | 11,993 | 1,374 |
| Example 6 | AlN | 6.3 | 20 | 2.1 | 0 | 7,878 | 1,052 |
| Comparative Example 1 | - | - | - | - | - | 5,826 | 627 |
| Comparative Example 2 | Cr | 0 | 20 | 4.2 | 4.15 | - | - |
| Comparative Example 3 | GaSb | 0.6 | 20 | 4.1 | 2.9×10⁻¹ | - | - |
| Comparative Example 4 | SiO₂ | 7.9 | 20 | 1.5 | 0 | 6,016 | 865 |

### <Evaluation>

As apparent from Table 1, the detection sensitivity of each of the SPR sensor cells of Examples is excellent as compared to Comparative Examples. More specifically, it is understood that the detection sensitivity of each of the SPR sensor cells of Examples in which the coat layer is formed of a material having a band gap (Eg) of more than 1.0 eV and less than 7.0 eV is remarkably excellent as compared to Comparative Example 1 in which no coat layer is formed and Comparative Example 4 in which a material having a band gap of more than 7.0 is used. This is probably because the refractive index of the coat layer of each of Examples is extremely large and its extinction coefficient in the near-infrared region is sufficiently small. In Comparative Example 2 in which the coat layer was formed with a metal, the extinction coefficient was so large that no SPR peak was able to be confirmed. In Comparative Example 3 in which a material having a band gap of less than 1.0 was used, the extinction coefficient was so large that no SPR peak was able to be confirmed.

### Industrial Applicability

The SPR sensor cell and SPR sensor of the present invention can be used suitably in various chemical analyses and biochemical analyses such as the measurement of a sample concentration and the detection of an immunoreaction.

### Reference Signs List

- **10**: detection unit
- **11**: under clad layer
- **12**: core layer
- **13**: protective layer
- **14**: metal layer
- **15**: coat layer
- **16**: over clad layer
- **20**: sample mounting portion
- **100**: SPR sensor cell
- **110**: light source
- **120**: light measuring device
- **200**: SPR sensor

## Claims

1. An SPR sensor cell, comprising:
a detection unit; and
a sample mounting portion adjacent to the detection unit,
wherein:
the detection unit includes an under clad layer, a core layer formed so that at least a part thereof is adjacent to the under clad layer, a metal layer covering the core layer, and a coat layer covering the metal layer;
the coat layer is formed of a material having a band gap (Eg) of more than 1.0 eV and less than 7.0 eV; and
a refractive index of the coat layer is higher than a refractive index of the core layer.

2. The SPR sensor cell according to claim 1, wherein the material for forming the coat layer comprises at least one material selected from silicon, zinc selenide, gallium arsenide, aluminum nitride, and N,N'-di(1-naphthyl)-N,N'-diphenylbenzidine.

3. The SPR sensor cell according to claim 1 or 2, wherein the refractive index of the coat layer is 1.60 or more.

4. The SPR sensor cell according to any one of claims 1 to 3, wherein the coat layer has an extinction coefficient of 1.80×10⁻² or less.

5. The SPR sensor cell according to any one of claims 1 to 4, wherein the coat layer has a thickness of 5 nm to 260 nm.

6. An SPR sensor, comprising the SPR sensor cell according to any one of claims 1 to 5.
